(19) Europäisches Patentamt / European Patent Office / Office européen des brevets

(11) **EP 3 378 481 B1**

(12) **EUROPEAN PATENT SPECIFICATION**

(45) Date of publication and mention
of the grant of the patent:
**05.04.2023 Bulletin 2023/14**

(21) Application number: **16865806.0**

(22) Date of filing: **21.11.2016**

(51) International Patent Classification (IPC):
*A61K 31/7048* (2006.01)   *A61P 5/50* (2006.01)
*A61P 3/10* (2006.01)   *A61P 1/18* (2006.01)
*A61K 31/045* (2006.01)   *A61K 31/047* (2006.01)
*A61K 31/353* (2006.01)   *A61K 31/428* (2006.01)
*A23L 33/10* (2016.01)

(52) Cooperative Patent Classification (CPC):
**A61K 31/045; A61K 31/047; A61K 31/353;
A61K 31/428; A61K 31/7048; A61P 1/18**

(86) International application number:
**PCT/CN2016/106607**

(87) International publication number:
**WO 2017/084631 (26.05.2017 Gazette 2017/21)**

(54) **COMPOUND OR COMPOSITION FOR PREVENTING OR TREATING PANCREAS FATTY INFILTRATION**

STOFF ODER ZUSAMMENSETZUNG ZUR PRÄVENTION ODER BEHANDLUNG VON BAUCHSPEICHELFETTINFILTRATION

COMPOSÉ OU COMPOSITION PHARMACEUTIQUE ET MÉTHODE DESTINÉES À PRÉVENIR OU TRAITER L'INFILTRATION GRAISSEUSE DU PANCRÉAS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.11.2015 US 201562257707 P**

(43) Date of publication of application:
**26.09.2018 Bulletin 2018/39**

(73) Proprietor: **Jacob Biotechnology Ltd.
Wenshan Dist., Taipei City 116064 (TW)**

(72) Inventors:
• **HU, Oliver Yoa-Pu
Taipei City 114
Taiwan (TW)**
• **HO, Hsin-Tien
Taipei City 114
Taiwan (TW)**
• **WU, Yung-En
Taipei City 114
Taiwan (TW)**
• **TANG, Hsi-Hui
Taipei City 114
Taiwan (TW)**

(74) Representative: **Straus, Alexander et al
2K Patent- und Rechtsanwälte - München
Keltenring 9
82041 Oberhaching (DE)**

(56) References cited:
EP-A1- 1 925 311   WO-A1-2010/044371
WO-A2-2011/045732   CN-A- 1 840 518
CN-A- 1 911 114   CN-A- 102 387 793
JP-A- 2008 007 452   JP-A- 2009 057 319
US-A- 5 578 623

EP 3 378 481 B1

- KODAMA H ET AL: "DIFFERENTIAL HYPOGLYCEMIC EFFECT OF 2,5-ANHYDRO-D-MANNITOL, A PUTATIVE GLUCONEOGENESIS INHIBITOR, IN GENETICALLY DIABETIC (DB/DB) AND STREPTOZOTOCIN-INDUCED DIABETIC MICE", JAPANESE JOURNAL OF PHARMACOL, THE JAPANESE PHARMACOLOGICAL SOCIETY, KYOTO, JP, vol. 66, no. 3, November 1994 (1994-11), pages 331-336, XP009040298, ISSN: 0021-5198, DOI: 10.1254/JJP.66.331
- WEI-YUN ZHANG ET AL: "Effect of Eriodictyol on Glucose Uptake and Insulin Resistance in Vitro", JOURNAL OF AGRICULTURAL AND FOOD CHEMISTRY, vol. 60, no. 31, 31 July 2012 (2012-07-31), pages 7652-7658, XP055592349, US ISSN: 0021-8561, DOI: 10.1021/jf300601z
- NICOLLE E ET AL: "Flavonoids as Promising Lead Compounds in Type 2 Diabetes Mellitus: Molecules of Interest and Structure-Activity Relationship", CURRENT MEDICINAL CHEMISTRY, BENTHAM, NL, vol. 18, no. 17, June 2011 (2011-06), pages 2661-2672, XP009177146, ISSN: 0929-8673, DOI: 10.2174/092986711795933777
- KIKUKO AMO ET AL: "Effects of xylitol on metabolic parameters and visceral fat accumulation", JOURNAL OF CLINICAL BIOCHEMISTRY AND NUTRITION., vol. 49, no. 1, 2011, pages 1-7, XP055592354, JP ISSN: 0912-0009, DOI: 10.3164/jcbn.10-111
- VARAYU PRACHAYAKUL ET AL: "Pancreatic Steatosis: What Should Gastroenterologists Know?", JOURNAL OF THE PANCREAS, vol. 16, no. 3, 20 May 2015 (2015-05-20), pages 227-231, XP055592373,
- MARK M. SMITS ET AL: "The clinical significance of pancreatic steatosis", NATURE REVIEWS / GASTROENTEROLOGY & HEPATOLOGY, vol. 8, no. 3, March 2011 (2011-03), pages 169-177, XP055592374, US ISSN: 1759-5045, DOI: 10.1038/nrgastro.2011.4
- XU, CHEN et al.: "Influence of Various Absorption Enhancers on the Hypoglycemic Effect of Insulin via Colon Delivery", CHINESE PHARMACOLOGICAL BULLETIN, vol. 20, no. 08, 31 August 2004 (2004-08-31), page 912, XP009511094,
- UNNO K ET AL: "Protection of brain and pancreas from high-fat diet: Effects of catechin and caffeine", PHYSIOLOGY AND BEHAVIOR, ELSEVIER SCIENCE LTD., OXFORD, GB, vol. 96, no. 2, 16 February 2009 (2009-02-16), pages 262-269, XP025872945, ISSN: 0031-9384, DOI: 10.1016/J.PHYSBEH.2008.10.009 [retrieved on 2008-10-17]

**Description**

**CROSS-REFERENCE TO RELATED APPLICATIONS**

This application claims priority to U.S. Provisional Patent Application No. 62/257,707, filed on Nov. 19, 2015.

**FIELD OF THE INVENTION**

The present invention relates to compounds and compositions for use in preventing or treating fatty pancreas or ameliorating a pancreas disease caused by fatty pancreas.

**BACKGROUND**

The pancreas is an organ having both exocrine and endocrine functions in the human body. The exocrine portion includes acini, which secrete a variety of digestive enzymes. The endocrine portion includes pancreatic islets, which secrete hormones, such as insulin, etc., and have a very important effect in maintaining the level of blood glucose. The accumulation of fat in the pancreas may affect the functions of the pancreas and further cause a pancreas disease, diabetes mellitus, or other related disorders. In particular, diabetes mellitus is a disease caused by abnormal glucose metabolism. There are two types of diabetes mellitus, including non-insulin dependent or adult type, also known as diabetes mellitus type II; and insulin dependent or infant type, also known as diabetes mellitus type 1. In general, diabetes mellitus type II usually occurs in adults, is highly associated with obesity, and can be controlled by appropriate exercise, diet and medications; while patients suffering from diabetes mellitus type I are mostly children and adolescents, who cannot produce enough insulin inside the body and must be administered insulin by injection to maintain life.

Therefore, there is still a need to find effective ingredients to reduce the content of pancreatic fat effectively in a subject and to improve the related disorders.

**SUMMARY OF THE INVENTION**

The present invention discloses that one or more compounds have the efficacies of reducing a pancreatic fat content, preventing or treating fatty pancreas, or ameliorating a pancreas disease caused by fatty pancreas, in a subject, wherein the compound is selected from the group consisting of criodictyol, menthol, sucralose, mannitol, saccharin, and any combinations thereof.

Therefore, in one aspect, the present invention provides a compound for use in reducing a pancreatic fat content, preventing or treating fatty pancreas, or ameliorating a pancreas disease caused by fatty pancreas, in a subject.

In some embodiments, the compound is selected from the group consisting of menthol, mannitol, eriodictyol, sucralose, and any combinations thereof.

In some embodiments, the compound is: (1) a combination of menthol, mannitol, and eriodictyol; (2) a combination of mannitol and eriodictyol; (3) a combination of mannitol and sucralose; (4) a combination of eriodictyol and sucralose; (5) a combination of menthol and mannitol; or (6) a combination of eriodictyol, mannitol, and sucralose.

In some embodiments, the compounds of the invention are useful in preventing or treating fatty pancreas, or ameliorating a pancreas disease caused by fatty pancreas. In some embodiments, the pancreas disease or related disorders include alcoholic fatty pancreas diseases and nonalcoholic fatty pancreas diseases, in which the alcoholic fatty pancreas diseases include, but are not limited to, alcoholic fatty steatopancreatitis and alcoholic pancreatitis, and the nonalcoholic fatty pancreas diseases include, but are not limited to, nonalcoholic fatty stcatopancreatitis, pancreatic lipomatosis, pancreatic steatosis, fatty pancreas, lipomatous pseudohypertrophy of pancreas, fatty replacement of pancreas, and fatty infiltration of pancreas (Nat. Rev. Gastroenterol. Hepatol. 8, 169-177 (2011); World. J Gastroenterol. 2006 Dec 14; 12(46): 7421-7427; and Alcohol Clin Exp Res. 1982 Winter; 6(1):117-21).

In some embodiments, the compounds of the invention may be manufactured into a drug, a food additive, or a healthy food.

In another aspect, the present invention provides a composition, which comprises a combination of any two or more compounds selected from the group consisting of eriodictyol, menthol, sucralose, mannitol, and saccharin.

In some embodiments, the composition of the present invention comprises a combination of any two or more compounds selected from the group consisting of menthol, mannitol, eriodictyol and sucralose.

In some embodiments, the composition of the present invention comprises a combination selected from the group consisting of (1) a combination of menthol, mannitol and eriodictyol; (2) a combination of mannitol and eriodictyol; (3) a combination of mannitol and sucralose; (4) a combination of eriodictyol and sucralose; (5) a combination of menthol and mannitol; and (6) a combination of eriodictyol, mannitol, and sucralose.

The present invention also discloses that one or more of the compounds as described herein have the efficacies of regulating pancreatic functions, in particular reducing pancreatic dysfunction due to fat accumulation, for example, against insulin resistance and reducing blood glucose.

## DETAILED DESCRIPTION OF THE EMBODIMENTS

Unless otherwise indicated, all technical and scientific terms used herein have the same meaning as commonly understood by those of ordinary skill in the art to which this invention pertains.

The term "a" or "an" as used herein refers to at least one (one or more) in quantity, unless otherwise specified.

The present invention discloses that one or more of the compounds described above have the effects in reducing a pancreatic fat content, preventing or treating fatty pancreas, or ameliorating a pancreas disease caused by fatty pancreas. Thus, the present invention provides the recited compound(s) for use in reducing a pancreatic fat content, preventing or treating fatty pancreas, or ameliorating a pancreas disease caused by fatty pancreas. The present invention also provides a composition for use in reducing a pancreatic fat content, preventing or treating tatty pancreas, or ameliorating a pancreas disease caused by fatty pancreas in a subject.

As used herein, "a pancreatic fat content" refers to the amount of fat that accumulates in the pancreas of a subject, including the generalized lipid, such as triglyceride (TG), cholesterol, etc. As used herein, "reducing a pancreatic fat content" generally refers to reducing the abnormal amount of pancreatic fat in a subject to a normal level or a level close to the normal, and may also refer to the comparison of the same subject before and after taking the medicament, wherein the amount of the pancreatic fat in the body of the subject after taking the medicament is lower than that of the subject before taking the medicament. The normal level of the content of the pancreatic fat can be obtained through assessment by measuring and collecting the content of pancreatic fat in a group of normal subjects. For example, for a population, the amount of a normal level of pancreatic fat accounts for 3% of the weight of the pancreas; when the weight of the fat in the pancreas exceeds 5% of the weight of the pancreas, it is considered an abnormal accumulation of fat; and therefore "reducing a pancreatic fat content" can refer to reduction of the amount of abnormal pancreatic fat in a subject to 3% of the weight of the pancreas (the above-mentioned content of pancreatic fat is a relative value and is exemplary, and may vary due to the group of subjects and other factors). As used herein, "fatty pancreas" may refer to the condition in which the content of pancreatic fat in a subject is higher than the normal level. Standard methods of analysis can be used to assess the content of pancreatic fat, which include, but are not limited to, ultrasound analysis, magnetic resonance imaging (MRI), magnetic resonance spectroscopy (MRS), computed tomography (CT), and pathological sectioning of the pancreas.

According to the present invention, said compounds include commonly used excipients and bionavonoids, which are readily available (e.g., commercially available) by those skilled in the art, and may be provided to reduce a pancreatic fat content, to prevent or treat fatty pancreas, or to improve a pancreas disease caused by fatty pancreas, in the subjects. In some embodiments, a pancreas disease or the disorders due to abnormal accumulation of pancreatic fat include alcoholic fatty pancreas and nonalcoholic fatty pancreas.

The term "treating" as used herein refers to the application or administration of a composition including one or more active agents to a subject afflicted with a disease, a symptom or conditions of the disease, or a progression of the disease, with the purpose to cure, heal. alleviate, relieve. alter, remedy, ameliorate, improve, or affect the disease, the symptoms or conditions of the disease, the disabilities induced by the disease, or the progression of the disease.

The term "preventing" as used herein refers to a prophylactic or preventive measure of a disease or a symptom or condition of the disease, including, but not limited to, application or administration of one or more active agents to the subject, who has not yet been diagnosed as a patient suffering from the disease or the symptom or condition of the disease yet, but is susceptible or having a tendency thereto for the purpose of avoiding, preventing, or delaying the occurrence of the disease or the symptom or condition of the disease.

As used herein, the terms "individual" or "subject" includes human or non-human animals, such as companion animals (e.g. dogs, cats, etc.), farm animals (e.g. cattle, sheep, pigs, horses, etc.), or experimental animals (e.g. rats, mice, guinea pigs, etc.).

As used herein, the term "effective amount" refers to the amount of active ingredient that produces a desired biological efficacy or medical effect in the treated subject, for example, reduction of the content of pancreatic fat in the subject, prevention or treatment of fatty pancreas, or amelioration of a pancreas disease caused by fatty pancreas.

For the purpose of delivery and absorption, a therapeutically effective amount of the active ingredient according to the present invention may be formulated into a pharmaceutical composition in a suitable form with a pharmaceutically acceptable carrier.

As used herein, "pharmaceutically acceptable" means that the carrier is compatible with the active ingredient in the composition, and preferably can stabilize said active ingredient and is safe to the individual receiving the treatment. Said carrier may be a diluent, vehicle, excipient. or matrix to the active ingredient. Some examples of appropriate excipients include lactose, dextrose, sucrose, sorbose, mannose, starch, Arabic gum, calcium phosphate, alginates. tragacanth gum, gelatin, calcium silicate, microcrystalline cellulose, polyvinyl pyrrolidone, cellulose, sterilized water, syrup, and methylcellulose. The composition may additionally comprise lubricants, such as talc, magnesium stearate, and mineral oil; wetting agents; emulsifying and suspending agents; preservatives, such as methyl and propyl hydroxybenzoates; sweeteners; and flavoring agents. The composition of the present invention can provide the effect of rapid, continued, or delayed release of the active ingredient after administration to the patient.

According to the present invention, the form of said composition may be tablets, pills, powder. lozenges, packets, troches, clixers, suspensions, lotions, solutions, syrups, soft and hard gelatin capsules, suppositories, sterilized injection fluid, and packaged powder.

The composition of the present invention may be delivered via any physiologically acceptable route, such as oral, parenteral (such as intramuscular, intravenous, subcutaneous, and intraperitoneal), transdermal, suppository, and intranasal methods. Regarding parenteral administration, it is preferably used in the form of a sterile water solution, which may comprise other substances, such as salts or glucose sufficient to make the solution isotonic to blood. The water solution may be appropriately buffered (preferably with a p11 value of 3 to 9) as needed. Preparation of an appropriate parenteral composition under sterile conditions may be accomplished with standard pharmacological techniques well known to persons skilled in the art.

The present invention also discloses that one or more compounds have the efficacy of regulating the functions of the pancreas, in particular to reduce the pancreatic dysfunction due to fat accumulation, such as the symptoms of diabetes. The efficacy of the compounds of the present invention includes counteraction of insulin resistance, reduction of blood glucose, and the like. The standard analyses may be used to assess pancreatic dysfunction and the symptoms of diabetes, which include, but are not limited to. blood glucose levels, glycated hemoglobin (HbAIC), glucose tolerance test (GTT), blood insulin concentration, homeostasis model assessment of insulin resistance (HOMA-IR), tumor necrosis factor-alpha analysis, interleukin-6 (IL-6) analysis, interleukin-1 beta (IL-1$\beta$) analysis, and the like.

Examples

1. Materials and methods

1.1 Test animals

The experimental animals employed were B6 mice. The number of test animals in each group of the pre-test was four or more, and the number of test animals in each group of the confirmation test was twelve or more. Male mice having a weight of 18 to 23 g were bred in an animal room with a normal light and dark cycle (7:00 am to 7:00 pm being the bright period, and the rest being the dark period) at a temperature of $23\pm2$ °C and a relative humidity of $55\pm15$ %. After the animals were purchased from Lesco Biotech Co., Ltd. (Taipei) and housed at the National Defense Animal Center, the animal experiments were conducted following the Experimental Guide of National Health Research Institute. First, the animals were fed with 3-5 grams of general diet daily, and the water was supplied infinitely. After 1 to 2 weeks of breeding, the healthy state was observed. The body weight was recorded once a week.

**1.2 Animal grouping**

The test animals were randomly grouped into blank control (Blank), high-fat control (HFD). positive control (PS), and test group. The blank control was given a normal diet; the high-fat control was given a high-fat diet; the positive control was given a high-fat diet and tube fed with silymarin (5 mg/kg/day) (Iranian Journal of Pharmaceutical Research (2016), 15 (3): 493-500; Life Sciences 75 (2004) 2167-2180; and Pharm Biol. 2016 Jul 8:1-6); and the test group was given a high-fat diet and tube fed with the test ingredient individually.

**1.3 Feeding method**

The blank control was fed with a normal diet arbitrarily for 12 weeks. The high-fat control, the positive control, and the test group were fed with a high-fat diet arbitrarily for 12 weeks. After 8 weeks of feeding, the blank control and the high-fat control were tube fed with deionized water once a day. The positive control was tube fed with silymarin once a day. The test group was tube fed with test compound(s) once a day for 4 weeks.

**1.4 Blood collection and various analyses**

Before the start of the test and at the 8th and 12th week after the start of the test, blood was collected from the check or heart. All the animals were weighed and then sacrificed at the end of the test, and blood was collected from the check or heart again. The blood samples of mice were allowed to stand at room temperature for 1 hour to coagulate, and then centrifuged at 15,700 x g by a frozen centrifuge at 4 °C for 5 minutes to separate the scrum.

### 1.4.1 Analysis of serum triglyceride (TG) and total cholesterol (TCHO, TC)

Triglyceride (TG) and total cholesterol (TCHO, TC) in the serum were measured by the automatic blood biochemical analyzer.

### 1.4.2 Analysis of biochemical functions of the pancreas: blood glucose analysis (GLU)

Blood glucose analysis was performed with Roche's ACCU-CI-ICK Active blood glucose meter. One day before blood collection, mice were fasted overnight. The code card of the blood glucose test paper was inserted into the blood glucose meter, and then the blood glucose test paper was inserted. After the blood drop pattern appeared on the meter, a drop of blood was taken from in vein at the cheek of the mouse with a buccal needle and then dropped into the blood glucose test paper. The blood glucose data shown on the blood glucose meter were recorded.

### 1.4.3 Analysis of biochemical functions of the pancreas: Intraperitoneal glucose tolerance test (IPGTT)

The mice were moved into a clean cage and fasted overnight. There was no food or feces at the feeding funnel or the bottom of the clean cage to ensure that the animals may get drinking water at all times. The body weight of the mice was measured, and the volume of the glucose solution required for intraperitoneone (IP) injection was calculated (based on a concentration of 250 mg/mL and 2 g of glucose per kg of mouse weight, the volume ($\mu$l) required would be 8x body weight (g)). The mice were moved out and injected intraperiloneally with an appropriate amount of glucose solution, and the injection time was recorded. Blood samples were taken from the tail of the mice at 0, 15. 30. 60, 120 and 150 minutes after the glucose solution was injected. The concentration of blood glucose was measured, and the blood glucose analysis was performed using the Roche's ACCU-CHEK Active blood glucose meter.

### 1.4.4 Analysis of biochemical functions of the pancreas: analysis of fasting insulin concentration

At the end of the test (at the twelfth week), the mice were fasted to collect the blood following the operation instructions of the mouse ultrasensitive insulin ELISA kit (ALPCO).

### 1.4.5 Analysis of biochemical functions of the pancreas: homeostasis model assessment of insulin resistance (HOMA-IR)

The mice were fasted to collect the blood at the end of the test (at the twelfth week), and the concentrations of blood glucose and insulin were measured and calculated according to the following formula:

$$\frac{\text{Conc. of fasting blood insulin (ng/mL) x Conc. of fasting blood glucose (mg/dL)}}{405}$$

### 1.4.6 Analysis of triglyceride (TG) and total cholesterol (TCHO, TC) in pancreatic tissue

After the mice were sacrificed, the pancreas was taken out and weighed to compare the ratios of pancreas weight to body weight. The remaining pancreas was frozen and preserved, and the contents of triglyceride and the total cholesterol in the pancreas were measured.

### 1.5 Statistical Analysis

All data were expressed as mean L standard deviation (SD), and the test results were calculated using the ANOVA test method to determine whether there was a statistically significant difference by using the Statistical Package of the Social Science program (Version 13, SPSS Inc.) software package. Subsequently, post hoc test least significant difference method was used to perform multiple comparisons to confirm the significant differences between the groups. The significant difference between the averages of the groups was $p < 0.05$.

## 2. Results

### 2.1 Animal experiments

In the animal experiments, all the animals were given high-fat diet, except that the blank control was given normal diet. After 8 weeks, in addition to the original diet, the animals in each group were further given different treatment for 4 weeks, in which the blank control and the high-fat control were given deionized water, the positive control was given silymarin, and the test group was given different test compounds, including pucrarin, phloridzin, eriodictyol, sucralose, mannitol, saccharin, hesperitin, menthol, or a combination of part of the test compounds. There was no statistically significant difference in weight gain and daily dietary intake of animals between these groups ($p > 0.05$).

In addition, after administration of different test compounds, the animals of the test group did not exhibit any abnormal symptoms, and no animal died during the test. After the test, the animals were sacrificed and anatomized for examination. No disease or clinical symptom caused by the test compounds was observed, and therefore, the test compounds are safe.

### 2.2 Test compounds have the effect of reducing pancreatic lipid

The results of the animal experiments showed that various test compounds exhibited the effect of reduction of the panceitic fat in the animals after 4 weeks of administration. The results are shown in Table 1.

Table 1. The test compounds reduced the pancreatic fat of the animals (4 weeks of administration).

| Item | pancreatic TG | pancreatic TC |
|---|---|---|
| Unit | mg/g pancreatic tissue | mg/g pancreatic tissue |
| Blank control (n=13) | 3.1±2.0 | 3.2±0.6 |
| High-fat control (n=12) | 34.6±38.0 | 6.1±3.5 |
| Positive control | | |
| Silymarin 5.0 mg/kg (n=6) | 15.8±18.8 * | 3.0±1.4 *** |
| silymarin 1.5 mg/kg (n=6) | 24.9±26.2 | 4.6±1.0 |
| single test compound | | |
| eriodictyol 2.5 mg/kg (n=6) | 4.5±2.8 *** | 3.5±0.6 *** |
| mannitol 7.5 mg/kg (n=6) | 4.7±4.0 *** | 3.1±0.3*** |
| mannitol 4.5 mg/kg (n=6) | 9.6±5.1 *** | 3.3±0.7 *** |
| mannitol 1.5 mg/kg (n=6) | 12.6±7.5 ** | 3.7±1.0 *** |
| saccharin 1.5 mg/kg (n=3) | 4.7±2.9 * | 3.0±0.8 ** |
| sucralose 7.5 mg/kg (n=6) | 5.2±2.1 *** | 3.1±0.4 *** |
| sucralose 1.5 mg/kg (n=6) | 8.1±5.6 *** | 4.2±0.5 * |
| hesperitin 2.5 mg/kg (n=6) | 5.7±1.6 *** | 4.4±0.7 *** |
| pucrarin 2.5 mg/kg (n=6) | 17.3±13.4 * | 4.0±1.2 * |
| menthol 1.5 mg/kg (n=6) | 18.1±18.3 * | 4.4±0.9 * |
| phloridzin 2.5 mg/kg (n=6) | 23.3±24.0 | 3.3±1.1 *** |
| A combination of two test compounds | | |
| Menthol + mannitol 1.5 mg/kg + 1.5 mg/kg (n=6) | 9.3±7.7 *** | |
| A combination of three test compounds | | |
| Menthol + mannitol + eriodictyol .5 mg/kg + .5 mg/kg + .8 mg/kg (n=6) | 7.5±3.6 *** | 5.4±2.1 |

The data represent mean ± standard deviation. Statistical differences using ANOVA and LSD are shown in the text. * p <0.05, ** p <0.01, *** p <0.005, compared with the high-fat control.

TG: triglyceride

TC: total cholesterol

The results showed that triglycerides and total cholesterol in the pancreas of mice given high-fat diet were increased. When a single test compound was administered, eriodictyol, mannitol, saccharin, sucralose, hesperitin, pucrarin, and menthol could significantly reduce about 33% - 87% of triglyceride in the pancreas, as compared with the high-fat control. In particular, 4 weeks of eriodictyol treatment achieved an excellent result. which reduced about 87% of the triglyceride content in the pancreas (p <0.005). In addition, eriodictyol, mannitol, saccharin, sucralose, hesperitin, pucrarin, menthol, and phloridzin could significantly reduce about 28% - 51% of the total cholesterol in the pancreas. In particular, 4 weeks of saccharin treatment achieved an excellent result, which reduced about 51% of the total cholesterol content in the pancreas (p <0.005).

When two or three compounds were administered in a combination, for example, a combination of menthol and mannitol or a combination of menthol, mannitol, and eriodictyol, in which the dosage of each ingredient was maintained at the same dosage as used alone or lower (e.g.. about one-third of the dosage as used alone), a similar or better effect of triglyceride reduction was achieved. Therefore, according to the present invention, the combination of particular test compounds can reduce the dosage, but maintain similar or better efficacy, resulting in a synergistic effect.

**2.3 Test compounds have the effect of regulating pancreatic functions and treating diabetes symptoms**

The results of animal experiments showed that various test compounds showed the regulation of pancreatic functions and reduction of pancreatic dysfunction in animals, in particular the function of carbohydrate metabolism, during 4 weeks of administration. The results are shown in Table 2-1.

Table 2-1. The test compounds can regulate pancreatic functions and reduce pancreatic dysfunction in animals (4 weeks of administration)

| Item | GLU | AUC of IPGTT |
|---|---|---|
| Unit | mg/dL | Hr*g/dL |
| Blank control (n=13) | 110.5±41.3 *** | 26.0±9.6 *** |
| High-fat control (n=12) | 198.7±45.6 | 53.7±11.1 |
| Positive control | | |
| Silymarin 5.0 mg/kg (n=6) | 210.3±35.9 | 53.1±4.8 |
| silymarin 1.5 mg/kg (n=6) | 121.0±28.6 *** | 41.8±6.5 *** |
| single test compound | | |
| phloridzin 2.5 mg/kg (n=6) | 216.2±26.4 | 51.3±8.9 |
| eriodictyol 2.5 mg/kg (n=6) | 219.0±35.6 | 41.9±5.9 *** |
| sucralose 1.5 mg/kg (n=3) | 128.7±19.3 *** | 44.2±3.7 * |
| menthol 1.5 mg/kg (n=6) | 125.7±33.5 *** | 45.6±4.8 * |
| mannitol 1.5 mg/kg (n=6) | 112.2±32.0 *** | 42.7±1.7 ** |
| saccharin 1.5 mg/kg (n=3) | 92.0±9.6 *** | 41.9±6.2 * |
| pucrarin 2.5 mg/kg (n=6) | 172.3±22.2 | 45.1±11.2 * |

The data represent mean ± standard deviation. Statistical differences using ANOVA and LSD are shown in the text. * p <0.05, ** p <0.01, *** p <0.005, compared with the high-fat control.

TG: triglyceride

TC: total cholesterol

The AUC of IPGTT represents the area under the curve of blood glucose concentration vs. time during the IPGTT test.

Table 2-2. The test compounds can regulate pancreatic functions and reduce pancreatic dysfunction in animals (4 weeks of administration).

| Item | fasting insulin concentration | HOMA-IR |
|---|---|---|
| Unit | mg/dL | |
| Blank control (n=13) | 0.6±0.1 *** | 0.2±0.1 *** |
| High-fat control (n=12) | 5.6±4.6 | 2.5±2.0 |
| Positive control | | |
| Silymarin 5.0 mg/kg (n=6) | 0.8±0.2 *** | 0.4±0.1 *** |
| silymarin 1.5 mg/kg (n=6) | 1.2±0.6 *** | 0.4±0.2 *** |
| single test compound | | |
| phloridzin 2.5 mg/kg (n=6) | 1.1±0.3 *** | 0.6±0.2 *** |
| eriodictyol 2.5 mg/kg (n=6) | 1.1±0.4 *** | 0.6±0.2 *** |
| sucralose 1.5 mg/kg (n=3) | 1.3±0.5 *** | 0.4±0.2 *** |
| menthol 1.5 mg/kg (n=6) | 1.2±0.8 *** | 0.4±0.4 *** |
| mannitol 1.5 mg/kg (n=6) | 1.1±0.4 *** | 0.3±0.1 *** |
| saccharin 1.5 mg/kg (n=3) | 1.3±1.1 *** | 0.3±0.3 *** |
| puerarin 2.5 mg/kg (n=6) | 0.9±0.3 *** | 0.4±0.2 *** |
| A combination of two test compounds | | |
| Menthol + mannitol .5 mg/kg + .5 mg/kg (n=6) | 0.7±0.2 *** | 0.2±0.1 *** |
| A combination of three test compounds | | |
| Menthol + mannitol + eriodictyol .5 mg/kg + .5 mg/kg + .8 mg/kg (n=6) | 1.1±0.6 *** | 0.4±0.2 *** |

The data represent mean ± standard deviation. Statistical differences using ANOVA and LSD are shown in the text. * p <0.05, ** p <0.01, *** p <0.005, compared with the high-fat control.

HOMA-IR: homeostasis model assessment of insulin resistance, calculated as the following formula:

Fasting serum insulin (ng/mL) x Fasting serum glucose (mg/dL) / 405

This experiment was performed to check whether the test compounds could adjust the functions of the pancreas or reduce the pancreatic dysfunction in animals. especially for the function of carbohydrate metabolism.

The results showed that various carbohydrate metabolism functions of the pancreas were impaired in the mice fed with high-fat diet (GLU, IPGTT. fasting insulin concentration, and HOMA-IR values increased). When single test compound was administered, sucralose, menthol, mannitol, and saccharin could significantly reduce GLU: eriodictyol, sucralose, menthol, mannitol, saccharin, and pucrarin could significantly reduce sugar tolerance; and phloridzin, eriodictyol, sucralose, menthol, mannitol, saccharin, and pucrarin could significantly reduce fasting insulin concentration and insulin resistance.

When two or three compounds were administered in a combination, for example, a combination of menthol and mannitol or a combination of menthol, mannitol, and eriodictyol, in which the dosage of each ingredient was lower than the dosage as used alone (e.g., about one-third of the dosage as used alone), a similar or better effect of reduction in fasting insulin concentration and insulin resistance was achieved (The fasting insulin concentration and HOMA-IR value were significantly reduced). Therefore, according to the present invention, it was shown that the combined use of particular test compounds can reduce the dosage, but still maintain similar or better efficacy, resulting in a synergistic effect.

## 2.4 Test compounds have the effect of reducing triglyceride in plasma

The results of animal experiments showed that various test compounds showed the reduction of plasma triglyceride in animals during 4 weeks of administration. The results arc shown in Table 3.

Table 3: The test compounds can reduce plasma triglyceride in animals (4 weeks of administration)

| Item | TG |
|---|---|
| Unit | mg/dL |
| Blank control (n=13) | 101.3±27.1 *** |
| High-fat control (n=12) | 185.4±34.8 |
| Positive control | |
| Silymarin 5.0 mg/kg (n=6) | 124.6±26.7 *** |
| silymarin 1.5 mg/kg (n=6) | 147.0±38.6 * |
| single test compound | |
| mannitol 7.5 mg/kg (n=6) | 163.9±31.9 |
| mannitol 4.5 mg/kg (n=6) | 180.1±46.7 |
| mannitol 1.5 mg/kg (n=6) | 158.9±48.9 |
| sucralose 7.5 mg/kg (n=6) | 186.9±17.2 |
| sucralose 1.5 mg/kg (n=6) | 126.9±43.3 *** |
| eriodictyol 5.0 mg/kg (n=6) | 143.2±15.1 * |
| eriodictyol 2.5 mg/kg (n=6) | 135.4±43.4 *** |
| puerarin 2.5 mg/kg (n=6) | 166.7±22.0 |
| phloridzin 2.5 mg/kg (n=6) | 148.3±31.4 * |
| hesperitin 2.5 mg/kg (n=6) | 190.1±25.9 |
| menthol 1.5 mg/kg (n=6) | 173.7±56.7 |
| saccharin 1.5 mg/kg (n=3) | 142.6±34.6 |
| A combination of two test compounds | |
| Menthol + mannitol .5 mg/kg + .5 mg/kg (n=6) | 155.7±27.6 |
| Menthol + mannitol 1.5 mg/kg + 1.5 mg/kg (n=6) | 120.2±9.0 *** |
| Sucralose + mannitol 7.5 mg/kg + 7.5 mg/kg (n=6) | 200.0±31.5 |
| Sucralose + mannitol 1.5 mg/kg + 1.5 mg/kg (n=6) | 230.5±32.9 |
| Eriodictyol + mannitol 5.0 mg/kg + 7.5 mg/kg (n=6) | 154.0±20.8 |
| Eriodictyol + sucralose 5.0 mg/kg + 7.5 mg/kg (n=6) | 115.1±9.2 *** |
| Saccharin + mannitol 1.5 mg/kg + 1.5 mg/kg (n=6) | 189.7±17.1 |
| A combination of three test compounds | |

| Menthol + mannitol + eriodictyol .5 mg/kg + .5 mg/kg + .8 mg/kg (n=6) | 150.9±34.3 * |
|---|---|

The data represent mean ± standard deviation. Statistical differences using ANOVA and LSD are shown in the text. * P <0.05, ** p <0.01, *** p <0.005, compared with the high-fat control.
TG: triglyceride

The results showed that feeding mice with high-fat diet induced increase of triglyceride in plasma. When a single test compound was administered, eriodictyol and phloridzin could significantly reduce triglyceride in plasma (the best being reduction by about 27%), as compared with the high-fat control.

When a combination of two test compounds was administered, the combination of menthol and mannitol or the combination of criodictyol and sucralose could significantly reduce triglyceride in plasma.

When a combination of three test compounds was administered, the combination of menthol, mannitol, and eriodictyol could effectively reduce triglyceride in plasma with a low dosage.

The compounds provided by the invention can reduce the content of pancreatic fat and improve the related disorders in a subject. The compounds belong to low molecular natural plant phenolic compounds, and are widely found in fruits and vegetables, grains, rhizomes. flowers, tea and red wine, etc. Through the animal tests, the compounds are confirmed to be safe, and have the potential to be developed into a healthy food or a medicament for reducing a pancreatic fat content, preventing or treating fatty pancreas, improving a pancreas disease caused by fatty pancreas.

## Claims

1. A compound selected from the group consisting of eriodictyol, menthol, sucralose, mannitol, saccharin, and any combinations thereof for use in reducing a pancreatic fat content, preventing or treating fatty pancreas, or ameliorating a pancreas disease caused by fatty pancreas.

2. The compound for use of claim 1, wherein the compound is selected from the group consisting of menthol, mannitol, eriodictyol, sucralose, and any combinations thereof.

3. The compound for use of claim 1, wherein the compound is: (1) a combination of menthol, mannitol and eriodictyol; (2) a combination of mannitol and eriodictyol; (3) a combination of mannitol and sucralose; (4) a combination of eriodictyol and sucralose; (5) a combination of menthol and mannitol; or (6) a combination of eriodictyol, mannitol, and sucralose.

4. The compound for use of any of claims 1 to 3, wherein the related disorders include alcoholic fatty pancreas and nonalcoholic fatty pancreas.

5. A composition, which comprises a combination of any two or more compounds selected from the group consisting of menthol, sucralose, mannitol, saccharin, and eriodictyol for use in reducing a pancreatic fat content, preventing or treating fatty pancreas, or ameliorating a pancreas disease caused by fatty pancreas.

6. The composition for use of claim 5, which comprises a combination of any two or more compounds selected from the group consisting of menthol, mannitol, eriodictyol and sucralose.

7. The composition for use of claim 5, which comprises a combination selected from the group consisting of (1) a combination of menthol, mannitol and eriodictyol; (2) a combination of mannitol and eriodictyol; (3) a combination of mannitol and sucralose; (4) a combination of eriodictyol and sucralose; (5) a combination of menthol and mannitol; and (6) a combination of eriodictyol, mannitol and sucralose.

8. The composition for use of any of claims 5 to 7, which is a drug, a food additive or a healthy food.

**Patentansprüche**

1. Verbindung, ausgewählt aus der Gruppe, bestehend aus Eriodictyol, Menthol, Sucralose, Mannitol, Saccharin und beliebigen Kombinationen davon, zur Verwendung bei der Verringerung des Fettgehalts der Bauchspeicheldrüse, der Vorbeugung oder Behandlung einer fettigen Bauchspeicheldrüse oder der Verbesserung einer durch eine fettige Bauchspeicheldrüse verursachten Erkrankung der Bauchspeicheldrüse.

2. Verbindung zur Verwendung nach Anspruch 1, wobei die Verbindung ausgewählt ist aus der Gruppe bestehend aus Menthol, Mannit, Eriodictyol, Sucralose und beliebigen Kombinationen davon.

3. Verbindung zur Verwendung nach Anspruch 1, wobei die Verbindung ist: (1) eine Kombination von Menthol, Mannit und Eriodictyol; (2) eine Kombination von Mannit und Eriodictyol; (3) eine Kombination von Mannit und Sucralose; (4) eine Kombination von Eriodictyol und Sucralose; (5) eine Kombination von Menthol und Mannit; oder (6) eine Kombination von Eriodictyol, Mannit und Sucralose.

4. Verbindung zur Verwendung nach einem der Ansprüche 1 bis 3, wobei die verwandten Störungen alkoholische Fettpankreas und nichtalkoholische Fettpankreas einschließen.

5. Zusammensetzung, die eine Kombination von zwei oder mehreren Verbindungen umfasst, ausgewählt aus der Gruppe bestehend aus Menthol, Sucralose, Mannit, Saccharin und Eriodictyol, zur Verwendung bei der Verringerung des Fettgehalts der Bauchspeicheldrüse, der Vorbeugung oder Behandlung einer fettigen Bauchspeicheldrüse oder der Verbesserung einer durch Bauchspeicheldrüsenfett verursachten Erkrankung der Bauchspeicheldrüse.

6. Zusammensetzung zur Verwendung nach Anspruch 5, die eine Kombination von zwei oder mehreren Verbindungen umfasst, ausgewählt aus der Gruppe bestehend aus Menthol, Mannit, Eriodictyol und Sucralose.

7. Zusammensetzung zur Verwendung nach Anspruch 5, die eine Kombination umfasst, ausgewählt aus der Gruppe bestehend aus (1) einer Kombination aus Menthol, Mannit und Eriodictyol; (2) einer Kombination von Mannit und Eriodictyol; (3) einer Kombination von Mannit und Sucralose; (4) einer Kombination von Eriodictyol und Sucralose; (5) einer Kombination von Menthol und Mannit; und (6) einer Kombination von Eriodictyol, Mannit und Sucralose.

8. Zusammensetzung zur Verwendung nach einem der Ansprüche 5 bis 7, die ein Arzneimittel, ein Lebensmittelzusatzstoff oder ein gesundes Lebensmittel ist.

**Revendications**

1. Composé choisi dans le groupe constitué par l'ériodictyol, le menthol, le sucralose, le mannitol, la saccharine et toute combinaison de ceux-ci, destiné à être utilisé pour réduire la teneur en graisse du pancréas, prévenir ou traiter la stéatose du pancréas ou améliorer une maladie du pancréas provoquée par la stéatose du pancréas.

2. Composé pour l'utilisation de la revendication 1, dans lequel le composé est choisi dans le groupe constitué par le menthol, le mannitol, l'ériodictyol, le sucralose, et toute combinaison de ceux-ci.

3. Composé pour l'utilisation de la revendication 1, dans lequel le composé est : (1) une combinaison de menthol, de mannitol et d'eriodictyol ; (2) une combinaison de mannitol et d'eriodictyol ; (3) une combinaison de mannitol et de sucralose ; (4) une combinaison d'eriodictyol et de sucralose ; (5) une combinaison de menthol et de mannitol ; ou (6) une combinaison d'eriodictyol, de mannitol et de sucralose.

4. Composé pour l'utilisation de l'une quelconque des revendications 1 à 3, dans lequel les troubles apparentés comprennent le pancréas gras alcoolique et le pancréas gras non alcoolique.

5. Composition, qui comprend une combinaison de deux ou plusieurs composés quelconques choisis dans le groupe constitué par le menthol, le sucralose, le mannitol, la saccharine et l'ériodictyol pour une utilisation dans la réduction de la teneur en graisse du pancréas, la prévention ou le traitement de la stéatose du pancréas, ou l'amélioration d'une maladie du pancréas provoquée par la stéatose du pancréas.

6. Composition à utiliser selon la revendication 5, qui comprend une combinaison de deux ou plusieurs composés quelconques choisis dans le groupe constitué par le menthol, le mannitol, l'eriodictyol et le sucralose.

7. Composition à utiliser selon la revendication 5, qui comprend une combinaison choisie dans le groupe constitué par (1) une combinaison de menthol, de mannitol et d'ériodictyol ; (2) une combinaison de mannitol et d'ériodictyol ; (3) une combinaison de mannitol et de sucralose ; (4) une combinaison d'ériodictyol et de sucralose ; (5) une combinaison de menthol et de mannitol ; et (6) une combinaison d'ériodictyol, de mannitol et de sucralose.

8. Composition à utiliser selon l'une quelconque des revendications 5 à 7, qui est un médicament, un additif alimentaire ou un aliment sain.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62257707

**Non-patent literature cited in the description**

- *Nat. Rev. Gastroenterol. Hepatol.,* 2011, vol. 8, 169-177
- *World. J Gastroenterol.,* 14 December 2006, vol. 12 (46), 7421-7427
- *Alcohol Clin Exp Res.,* 1982, vol. 6 (1), 117-21
- *Iranian Journal of Pharmaceutical Research,* 2016, vol. 15 (3), 493-500
- *Life Sciences,* 2004, vol. 75, 2167-2180
- *Pharm Biol.,* July 2016, vol. 8, 1-6